# EUROPEAN PATENT SPECIFICATION

(11) **EP 1 487 801 B1**
(45) Date of publication and mention of the grant of the patent: **19.04.2006**
(21) Application number: 03704631.5
(22) Date of filing: 13.02.2003
(51) Int. Cl.: C07D 223/16, C07D 409/12, A61K 31/55, A61P 3/04, A61P 25/06, A61P 25/08, A61P 25/22, A61P 25/24, A61P 25/28, A61P 25/30

(54) **7-ARYLSULFONAMIDO-2,3,4,5-TETRAHYDRO-1H-BENZO¬D|AZEPINE DERIVATIVES WITH 5-HT6 RECEPTOR AFFINITY FOR THE TREATMENT OF CNS DISORDERS**
7-ARYLSULFONAMID-2,3,4,5-TETRAHYDRO-1H-BENZO¬D|AZEPINE MIT 5-HT6 REZEPTOR AFFINITÄT ZUR BEHANDLUNG VON ZENTRALNERVENSYSTEMERKRANKUNGEN
DERIVES DE 7-ARYLSULFONAMIDO-2,3,4,5-TETRAHYDRO-1H-BENZO'DIAZEPINE PRESENTANT UNE AFFINITE POUR LE RECEPTEUR 5-HT6, DESTINES AU TRAITEMENT DE TROUBLES DU SNC

(30) Priority: 13.02.2002 GB 0203437; 28.02.2002 GB 0204758; 30.05.2002 GB 0212548; 23.08.2002 GB 0219711
(43) Date of publication of application: 22.12.2004
(73) Proprietor: GLAXO GROUP LIMITED, Greenford, Middlesex UB6 0NN (GB)
(72) Inventor: BROMIDGE, Steven Mark, 37135 Verona (IT); JOHNSON, Christopher Norbert, GlaxoSmithKline, Harlow, Essex CM19 5AW (GB); MOSS, Stephen Frederick, GlaxoSmithKline, Harlow, Essex CM19 5AW (GB); RAHMAN, Shahzad Sharooq, GlaxoSmithKline, Harlow, Essex CM19 5AW (GB); WITTY, David R, GlaxoSmithKline, Harlow, Essex CM19 5AW (GB)
(74) Representative: Gibson, Mark
(86) International application number: PCT/EP2003/001543
(87) International publication number: WO 2003/068751

(56) References cited:
- WO-A-01/32646
- WO-A-99/02502
- WO-A-02/089811
- US-A- 5 939 451

## Description

This invention relates to novel sulfonamide compounds having pharmacological activity, processes for their preparation, to compositions containing them and to their use in the treatment of CNS and other disorders.

WO 98/27081, WO 99/02502, WO 99/37623, WO 99/42465 and WO 01/32646 (SmithKline Beecham plc) disclose a series of aryl sulphonamide and sulphoxide compounds that are said to be 5-HT₆ receptor antagonists and which are claimed to be useful in the treatment of various CNS disorders. US-A-5 939 451 (Hoffman-La Roche) discloses a series of benzenesulfonamide compounds that are said to possess a selective affinity to 5-HT₆ receptors and accordingly are said to be useful for the treatment or prevention of Alzheimer's Disease.

A structurally novel class of compounds has now been found which possess affinity for the 5-HT₆ receptor. The present invention therefore provides, in a first aspect, a compound of formula (I) or a pharmaceutically acceptable salt thereof: 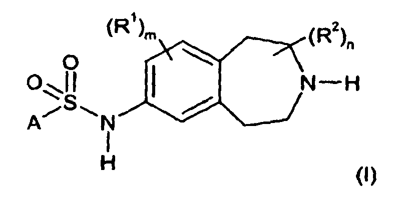 wherein:
R¹ represents hydrogen, halogen, hydroxy, cyano, nitro, trifluoromethyl, trifluoromethoxy, C₁₋₆ alkyl, trifluoromethanesulfonyloxy, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, arylsulfonyl, arylsulfonyloxy, arylsulfonylC₁₋₆ alkyl, C₁₋₆ alkylsulfonamido, C₁₋₆ alkylamido, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aroyl, aroylC₁₋₆ alkyl, arylC₁₋₆ alkanoyl, or a group CONR³R⁴ or SO₂NR³R⁴, wherein R³ and R⁴ independently represent hydrogen or C₁₋₆ alkyl or together may be fused to form a 5- to 7- membered aromatic or non-aromatic heterocyclic ring optionally interrupted by an O or S atom;
R² represents hydrogen or C₁₋₆ alkyl;
m represents an integer from 1 to 3;
n represents an integer from 1 to 4;
A represents phenyl, naphthyl or a monocyclic or bicyclic heteroaryl group each of which may be optionally substituted by one or more substituents which may be the same or different, and which are selected from those defined for R¹; or solvates thereof;
with the proviso that when A represents phenyl substituted at the 4-position, said substituent is not trifluoromethyl, trifluoromethoxy, C₃₋₆ alkyl or C₁₋₆ alkoxy.

Further specific groups of compounds of formula (I) which may be mentioned are those as defined above wherein m represents 0.

Alkyl groups, whether alone or as part of another group, may be straight chain or branched and the groups alkoxy and alkanoyl shall be interpreted similarly. Alkyl moieties are more preferably C₁₋₄ alkyl, eg. methyl or ethyl. The term 'halogen' is used herein to describe, unless otherwise stated, a group selected from fluorine, chlorine, bromine or iodine.

The term "aryl" includes phenyl and naphthyl.

The term "heteroaryl" is intended to mean a 5 or 6 membered monocyclic aromatic or a fused 8-10 membered bicyclic aromatic ring containing 1 to 3 heteroatoms selected from oxygen, nitrogen and sulphur. Suitable examples of such monocyclic aromatic rings include thienyl, furyl, pyrrolyl, triazolyl, imidazolyl, oxazolyl, thiazolyl, oxadiazolyl, isothiazolyl, isoxazolyl, thiadiazolyl, pyrazolyl, pyrimidyl, pyridazinyl, pyrazinyl and pyridyl. Suitable examples of such fused aromatic rings include benzofused aromatic rings such as quinolinyl, isoquinolinyl, quinazolinyl, quinoxalinyl, cinnolinyl, naphthyridinyl, indolyl, indazolyl, pyrrolopyridinyl, benzofuranyl, benzothienyl, benzimidazolyl, benzoxazolyl, benzisoxazolyl, benzothiazolyl, benzisothiazolyl, benzoxadiazolyl, benzothiadiazolyl and the like. Heteroaryl groups, as described above, may be linked to the remainder of the molecule via a carbon atom or, when present, a suitable nitrogen atom except where otherwise indicated above.

Preferably, A is substituted by 0 to 3 substituents, more preferably 0, 1 or 2 substituents. Preferably, A represents phenyl or a monocyclic heteroaryl group (such as thienyl) optionally substituted by one or more halogen (such as chlorine or bromine, eg. 3-chlorophenyl, 4-chlorophenyl, 4-bromophenyl, 5-bromophenyl, 2,3-dichlorophenyl or 3,5-dichlorophenyl), C₁₋₆ alkyl (such as methyl, eg. 4-methyl or ethyl, eg. 2-ethyl), C₁₋₆ alkoxy (such as methoxy, eg. 2-methoxy), trifluoromethoxy (such as 2-trifluoromethoxy) or trifluoromethyl (such as 3-trifluoromethyl) groups.
More preferably, A represents unsubstituted phenyl or phenyl substituted by 3-trifluoromethyl, halogen (eg. 4-chloro and 4-bromo) and/or 2-trifluoromethoxy.
Most preferably, A represents phenyl di-substituted by halogen (especially 4-chloro or 4-bromo) and 2-trifluoromethoxy.
Preferably, m is 0 or 1, most preferably 0.
When m is 1, R¹ is preferably halogen (eg. 6-chlorine, 9-chlorine or 9-bromine) or C₁₋₆ alkoxy (such as methoxy, eg. 8-methoxy).
Preferably, n is 0.

Preferred compounds according to the invention include examples E1-E17 as shown below, or a pharmaceutically acceptable salt thereof.

The compounds of formula (I) can form acid addition salts thereof. It will be appreciated that for use in medicine the salts of the compounds of formula (I) should be pharmaceutically acceptable. Suitable pharmaceutically acceptable salts will be apparent to those skilled in the art and include those described in J. Pharm. Sci., 1977, 66, 1-19, such as acid addition salts formed with inorganic acids e.g. hydrochloric, hydrobromic, sulfuric, nitric or phosphoric acid; and organic acids e.g. succinic, maleic, acetic, fumaric, citric, tartaric, benzoic, p-toluenesulfonic, methanesulfonic or naphthalenesulfonic acid. The present invention includes within its scope all possible stoichiometric and non-stoichiometric forms. Preferably, the compound of formula (I) forms an acid addition salt with hydrochloric acid.

The compounds of formula (I) may be prepared in crystalline or non-crystalline form, and, if crystalline, may optionally be solvated, eg. as the hydrate. This invention includes within its scope stoichiometric solvates (eg. hydrates) as well as compounds containing variable amounts of solvent (eg. water).

Certain compounds of formula (I) are capable of existing in stereoisomeric forms (e.g. diastereomers and enantiomers) and the invention extends to each of these stereoisomeric forms and to mixtures thereof including racemates. The different stereoisomeric forms may be separated one from the other by the usual methods, or any given isomer may be obtained by stereospecific or asymmetric synthesis. The invention also extends to any tautomeric forms and mixtures thereof.

The present invention also provides a process for the preparation of a compound of fonnula (I) or a pharmaceutically acceptable salt thereof, which process comprises:
(a) reacting a compound of formula (II) 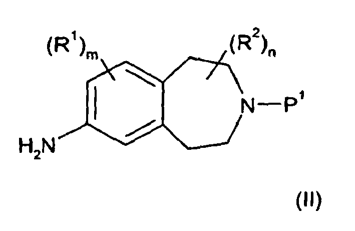 wherein R¹, R², m and n are hereinbefore defined and P' is a suitable protecting group such as acetyl, trifluoroacetyl, *t*-butyloxycarbonyl, 2',2',2'-trichloroethoxycarbonyl, benzyl or methyl, with a compound of formula (III) 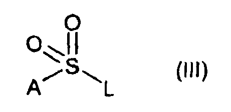 wherein A is as hereinbefore defined and L is a suitable leaving group, such as a halogen atom (eg. fluorine or chlorine) and thereafter deprotecting the resultant compound; or
(b) deprotecting a compound of formula (I) which is protected; and optionally thereafter
(c) interconversion to other compounds of formula (I).

Process (a) typically comprises the use of a suitable base such as pyridine or triethylamine in an inert solvent such as dichloromethane or tetrahydrofuran.

In process (b), examples of protecting groups and the means for their removal can be found in T. W. Greene 'Protective Groups in Organic Synthesis' (J. Wiley and Sons, 1991). Suitable amine protecting groups include sulphonyl (e.g. tosyl), acyl (e.g. acetyl, 2',2',2'-trichloroethoxycarbonyl, benzyloxycarbonyl or t-butoxycarbonyl) and arylalkyl (e.g. benzyl), which may be removed by hydrolysis (e.g. using an acid such as hydrochloric acid) or reductively (e.g. hydrogenolysis of a benzyl group or reductive removal of a 2',2',2'-trichloroethoxycarbonyl group using zinc in acetic acid) as appropriate. Other suitable amine protecting groups include trifluoroacetyl (-COCF₃) which may be removed by base catalysed hydrolysis or a solid phase resin bound benzyl group, such as a Merrifield resin bound 2,6-dimethoxybenzyl group (Ellman linker), which may be removed by acid catalysed hydrolysis, for example with trifluoroacetic acid.

Process (c) may be performed using conventional interconversion procedures such as epimerisation, oxidation, reduction, alkylation, nucleophilic or electrophilic aromatic substitution, ester hydrolysis or amide bond formation. Examples of process (c) include interconversions of the groups R¹, R² and A.

Compounds of formula (II) and (III) are known in the literature or can be prepared by analogous methods.

Pharmaceutically acceptable salts may be prepared conventionally by reaction with the appropriate acid or acid derivative.

Compounds of formula (I) and their pharmaceutically acceptable salts have affinity for the 5-HT₆ receptor and are believed to be of potential use in the treatment of certain CNS disorders such as anxiety, depression, epilepsy, obsessive compulsive disorders, migraine, cognitive memory disorders (e.g. Alzheimers disease, age related cognitive decline and mild cognitive impairment), Parkinsons Disease, ADHD (Attention Deficit Disorder/Hyperactivity Syndrome), sleep disorders (including disturbances of Circadian rhythm), feeding disorders such as anorexia and bulimia, panic attacks, withdrawal from drug abuse such as cocaine, ethanol, nicotine and benzodiazepines, schizophrenia, and also disorders associated with spinal trauma and/or head injury such as hydrocephalus. Compounds of the invention are also expected to be of use in the treatment of certain GI (gastrointestinal) disorders such as IBS (Irritable Bowel Syndrome). Compounds of the invention are also expected to be of use in the treatment of obesity.

Thus the invention also provides a compound of formula (I) or a pharmaceutically acceptable salt thereof, for use as a therapeutic substance, in particular in the treatment or prophylaxis of the above disorders. In particular the invention provides for a compound of formula (I) or a pharmaceutically acceptable salt thereof, for use in the treatment of depression, anxiety, obesity and cognitive memory disorders

In another aspect, the invention provides the use of a compound of formula (I) or a pharmaceutically acceptable salt thereof in the manufacture of a medicament for use in the treatment or prophylaxis of the above disorders.

In order to use the compounds of formula (I) in therapy, they will normally be formulated into a pharmaceutical composition in accordance with standard pharmaceutical practice. The present invention also provides a pharmaceutical composition, which comprises a compound of formula (I) or a pharmaceutically acceptable salt thereof, and a pharmaceutically acceptable carrier.

A pharmaceutical composition of the invention, which may be prepared by admixture, suitably at ambient temperature and atmospheric pressure, is usually adapted for oral, parenteral or rectal administration and, as such, may be in the form of tablets, capsules, oral liquid preparations, powders, granules, lozenges, reconstitutable powders, injectable or infusable solutions or suspensions or suppositories. Orally administrable compositions are generally preferred.

Tablets and capsules for oral administration may be in unit dose form, and may contain conventional excipients, such as binding agents, fillers, tabletting lubricants, disintegrants and acceptable wetting agents. The tablets may be coated according to methods well known in normal pharmaceutical practice.

Oral liquid preparations may be in the form of, for example, aqueous or oily suspension, solutions, emulsions, syrups or elixirs, or may be in the form of a dry product for reconstitution with water or other suitable vehicle before use. Such liquid preparations may contain conventional additives such as suspending agents, emulsifying agents, non-aqueous vehicles (which may include edible oils), preservatives, and, if desired, conventional flavourings or colourants.

For parenteral administration, fluid unit dosage forms are prepared utilising a compound of the invention or pharinaceutically acceptable salt thereof and a sterile vehicle. The compound, depending on the vehicle and concentration used, can be either suspended or dissolved in the vehicle. In preparing solutions, the compound can be dissolved for injection and filter sterilised before filling into a suitable vial or ampoule and sealing. Advantageously, adjuvants such as a local anaesthetic, preservatives and buffering agents are dissolved in the vehicle. To enhance the stability, the composition can be frozen after filling into the vial and the water removed under vacuum. Parenteral suspensions are prepared in substantially the same manner, except that the compound is suspended in the vehicle instead of being dissolved, and sterilization cannot be accomplished by filtration. The compound can be sterilised by exposure to ethylene oxide before suspension in a sterile vehicle. Advantageously, a surfactant or wetting agent is included in the composition to facilitate uniform distribution of the compound.

The composition may contain from 0.1% to 99% by weight, preferably from 10 to 60% by weight, of the active material, depending on the method of administration.

The dose of the compound used in the treatment of the aforementioned disorders will vary in the usual way with the seriousness of the disorders, the weight of the sufferer, and other similar factors. However, as a general guide suitable unit doses may be 0.05 to 1000 mg, more suitably 0.05 to 200 mg, for example 20 to 40 mg; and such unit doses will preferably be administered once a day, although administration more than once a day may be required; and such therapy may extend for a number of weeks or months.

The following Descriptions and Examples illustrate the preparation of compounds of the invention.

### Description 1

### 3-t-Butyloxycarbonyl-7-nitro-2,3,4,5-tetrahydro-1H-benzo[d]azepine (D1)

A solution of di-*t*-butyl dicarbonate (19.3g, 88.2mmol) in dichloromethane (150ml) was added over 0.5h to a stirred, ice-cooled solution of 7-nitro-2,3,4,5-tetrahydro-1*H*-benzo[*d*]azepine (11.3g, 58.8mmol) (for synthesis see Pecherer *et al*., *J.Het. Chem*., 1971, **8**, 779) and triethylamine (12.3ml, 88.2mmol) in dichloromethane (150ml) under argon. The solution was warmed to ambient temperature and stirred for 18h. The reaction mixture was then washed with water (2 x 400ml), dried (MgSO₄) and concentrated *in vacuo* to an oily solid. The solid was purified by chromatography over silica gel eluting with a solvent gradient of ethyl acetate/hexane to afford the title compound (D1) as a colourless solid (9.1g, 31.1mmol, 53%). δH (CDCl₃, 400MHz) 1.49 (9H, s), 3.00 (4H, br s), 3.58 (4H, br s), 7.27-7.29 (1H, br d), 7.98- 8.00 (2H, br, m).

### Description 2

### 7-Amino-3-t-butyloxycarbonyl-2,3,4,5-tetra6ydro-1H-benzo[d]azepine (D2)

A solution of 3-*t*-butyloxycarbonyl-7-nitro-2,3,4,5-tetrahydro-1*H*-benzo[*d*]azepine (D1) (8.0g, 27.4mmol) in ethanol (250ml) was stirred with 10% palladium on carbon (1.3g) for 20h under one atmosphere of hydrogen at ambient temperature. The reaction mixture was filtered to remove the catalyst and the filtrate was evaporated *in vacuo* to yield the title compound (D2) as a colourless oil (7.0g, 26.6mmol, 97%). δH (CDCl₃, 400MHz) 1.48 (9H, s), 2.78 (4H, br s), 3.51 (6H, br s), 6.45-6.48 (2H, m), 6.89 (1H, d, J = 7.5Hz).

### Description 3

### 3-t-Butyloxycarbonyl-7-(3-trifluoromethyl)phenylsulfonamido-2,3,4,5-tetrahydro-1H-benzo[d]azepine (D3)

A solution of 7-amino-3-*t*-butyloxycarbonyl-2,3,4,5-tetrahydro-1*H*-benzo[*d*]azepine (D2) (512mg, 1.95mmol) in dichloromethane (20ml) was treated successively with pyridine (1ml) and 3-trifluoromethylbenzenesulfonyl chloride [Lancaster] (2.15 mmol, 520mg) with stirring. After 2 hours, water (1ml) was added, and the mixture stirred vigorously for a further 2h then the solvents evaporated. The residue was dissolved in ethyl acetate (100ml) and washed successively with 5% aq. citric acid (50ml), water (50ml) and brine (50ml) then dried (MgSO₄) and evaporated *in vacuo*. The residue was purified by flash chromatography (gradient of ethyl acetate/hexane) on silica gel to afford the title compound (D3) as a clear oil (910mg, 99%). δH (CDCl₃, 400MHz) 1.47 (9H, s), 2.77-2.83 (4H, m), 3.48-3.49 (4H, m), 6.8 (1H, br s), 6.9 (1H, br s), 6.99 (1H,d), 7.23 (1H, s), 7.60 (1H, app.t), 7.79 (1H, d), 7.95 (1H, d), 7.98 (1H, s).

### Description 4

### 3-t-Butyloxycarbonyl-7-phenylsulfonamido-2,3,4,5-tetrahydro-1H-benzo[d]azepine (D4)

The title compound (D4) was prepared in 72% yield as described in Description 3, by treatment of 7-amino-3-*t*-butyloxycarbonyl-2,3,4,5-tetrahydro-1*H*-benza[*d*]azepine (D2) with phenyl sulfonyl chloride.
δH (D6-DMSO), 400MHz) 1.37 (9H, s), 2.70 (4H, br, s), 3.36 (4H, br, s), 6.83-6.85 (2H, m), 6.97 (1H, d, J = 8.3 Hz), 7.52-7.60 (3H, m), 7.75 (2H, d, J = 7.2Hz), 10.2 (1H, s).

### Description 5

### 7-Nitro-3-trifluoroacetyl-2,3,4,5-tetrahydro-1H-benzo[d]azepine (D5)

To a stirred, ice-cooled solution of 7-nitro-2,3,4,5-tetrahydro-1*H*-benzo[*d*]azepine (29g, 0.15mol) (for synthesis see Pecherer *et al., J.Het. Chem.,* 1971, 8, 779) in dichloromethane (1 litre) was slowly added triethylamine (41.8ml, 0.30mol) followed by dropwise addition of trifluoroacetic anhydride (42.4ml, 0.30mol). The resulting mixture was allowed to stir and warm to ambient temperature over 18h before being poured onto ice. The organic phase was separated and the aqueous layer extracted with dichloromethane (200ml). The combined organic phases were then washed with saturated aqueous sodium hydrogen carbonate (600ml), brine (600ml) and then dried (MgSO₄) and evaporated *in vacuo.* The crude residue (59g) was purified by column chromatography on silicagel eluting with ethyl acetate/ hexane (1:4) to afford the title compound (D5) as a yellow solid (37g, 0.128mol, 86%).
δH (CDCl₃, 400MHz) 3.08-3.13 (4H, m), 3.74-3.84 (4H, m), 7.31-7.36 (1H, m), 8.03- 8.07 (2H, m).

### Description 6

### 7-Amino-3-trifluoroacetyl-2,3,4,5-tetrahydro-1H-benzo[d]azepine (D6)

A mixture of 7-nitro-3-trifluoroacetyl-2,3,4,5-tetrahydro-1*H*-benzo[*d*]azepine (D5) (37g, 0.128mmol) and 10% palladium on carbon (5g) in ethanol (200ml) and 1,4-dioxane (600ml) was stirred with hydrogen at atmospheric pressure and room temperature for 18h. The mixture was filtered and the filtrate concentrated *in vacuo* to a solid which was stirred with hexane (200ml) for 1h. Filtration of the mixture gave the title compound (D6) as a solid (32.5g, 0.126mmol, 98%).
δH (CDCl₃, 400MHz) 2.84-2.89 (4H, m), 3.05 (2H, br, s), 3.63-3.68 (2H, m), 3.71-3.76 (2H, m), 6.48-6.51 (2H, m), 6.91-6.95 (1H, m).

### Description 7

### 7-(4-Bromo-2-trifluoromethoxy)phenylsulfonamido-3-t-butyloxycarbonyl-2,3,4,5-tetrahydro-1H-benzo[d]azepine (D7)

To a stirred solution of 7-amino-3-*t*-butyloxycarbonyl-2,3,4,5-tetrahydro-1*H*-benzo[*d*]azepine (D2) (23.4g, 89.3mmol) in pyridine (60ml) and dichloromethane (140ml) at 5°C was added 2-trifluoromethoxy-4-bromo-benzenesulfonyl chloride (33.4g, 98.2mmol) in dichloromethane (20ml) over 30 mins. The solution was then stirred at room temperature for 18 hours. The solvents were removed and the residue purified by chromatography on silica using 20% ethyl acetate in hexane. The crude product was recrystallised from ethyl acetate / hexane to afford the title compound (D7) as a pale yellow solid (30.5g, 60%).
δH (CDCl₃, 400MHz), 1.46 (9H, s), 2.79 (4H, m), 3.48 (4H, m), 6.63 (1H, s), 6.80 (1H, br s), 6.84 (1H, s), 6.96 (1H, d, J=8.08 Hz), 7.47 (1H, dd J=8.4, 1.6 Hz), 7.52 (1H, s), 7.79 (1H, d J=8.4Hz).

### Description 8

### 7-(4-Bromo-1-trifluoromethoxy)phenylsulfonamido-3-trifluoroacetyl-2,3,4,5-tetrahydro-1H-benzo[d]azepine (D8)

A solution of 4-bromo-2-trifluoromethoxybenzene sulfonyl chloride (42.4g, 125mmol) in dichloromethane (100ml) was added over 20 minutes to a stirred solution of 7-amino-3-trifluoroacetyl-2,3,4,5-tetrahydro-1*H*-benzo[*d*]azepine (D6) (32.3g, 125mmol) and pyridine (30.2ml, 375mmol) in dichloromethane (150ml) at room temperature under argon. After 18h the solution was diluted with dichloromethane (750ml) and washed successively with water (1 litre), 1M hydrochloric acid (2 x 1 litre) and brine (1 litre). The organic extract was dried (MgSO₄) and concentrated *in vacuo* to a red oil which was purified by column chromatography over silicagel eluting with a gradient of ethyl acetate/hexane to afford the title compound (D8) as a colourless oil (56g, 100mmol, 80%)
δH (CDCl₃, 400MHz) 2.87-2.91 (4H, m), 3.62-3.65 (2H, m), 3.70-3.73 (2H, m), 6.67 (1H, br, s), 6.83-6.86 (1H, m), 6.91 (1H, d, J = 2.2Hz), 6.99-7.03 (1H, m), 7.48-7.58 (2H, m), 7.79-7.82 (1H, m).
Found [MH]⁺ 561/563 (C₁₉H₁₅BrF₆N₂O₄S)

### Description 9

### 7-(4-Chloro-2-trifluoromethoxy)phenylsulfonamido-3-trifluoroacetyl-2,3,4,5-tetrahydra-1H-benzo[d]azepine (D9)

An efficiently stirred mixture of 7-(4-bromo-2-trifluoromethoxy)phenylsulfonamido-3-trifluoroacetyl-2,3,4,5-tetrahydro-1*H*-benzo[*d*]azepine (D8) (55g, 98mmol) and copper (I) chloride (193g, 1.94mol) in dry N,N-dimethylformamide (540ml) was heated at 120°C for 24h under argon. The mixture was cooled to ambient temperature then the solid filtered, and washed with dry *N,N*-dimethylformamide (2 x 100ml). To the filtrate and washings was added fresh copper (I) chloride (48g, 485mmol) and the stirred mixture reheated at 120°C for 18h under argon. The mixture was cooled to ambient temperature and the solid was filtered and washed with dichloromethane (6 x 200ml). The filtrate and washings were concentrated *in vacuo* and the residue re-concentrated with toluene (2 x 750ml). The residue was then stirred with dichloromethane (250ml) and the whole mixture filtered through kieselguhr. The filtrate was concentrated *in vacuo* and the residue partially purified by column chromatography over silicagel eluting with dichloromethane to afford crude title product (D9) as a foam (42.8g). This material was crystallised by dissolving in diethyl ether (150ml) at room temperature and adding hexane (150ml) with stirring and leaving to fully crystallize for 2 days. The colourless crystals were filtered off and identified as the title compound (D9) (24g, 46.4mmol, 47%). A second crop of the product (D9) (7.4g, total yield = 31.4g, 60.7mmol, 62%) was isolated from the filtrate by concentrating the filtrate and re-chromatographing the residue over silicagel (acetone/toluene gradient) followed by crystallisation from diethyl ether / hexane.
δH (CDCl₃, 400MHz) 2.87-2.91 (4H, m), 3.62-3.65 (2H, m), 3.70-3.73 (2H, m), 6.71 (1H, s), 6.83-6.87 (1H, m), 6.90 (1H, d, J = 2.3Hz), 6.99-7.03 (1H, m), 7.31-7.39 (2H, m), 7.87-7.90 (1H, m).
Found [MH]⁺ 517/519 (C₁₉H₁₅ClF₆N₂O₄S)

### Description 10

### 7-Amino-6-chloro-1,2,4,5-tetrahydro-benzo[d]azepine-3-carboxylic acid tert-butyl ester (D10)

7-Amino-3-*t*-butyloxycarbonyl-2,3,4,5-tetrahydro-1H-benzo[*d*]azepine (D2) (1.57g, 6mmol) was dissolved in acetonitrile (30ml) and treated with N-chlorosuccinimide (895mg, 6.5mmol) at 0°C and the stirred mixture allowed to warm to room temperature for 14 hours. Saturated aqueous sodium sulphite (5ml) was added and the mixture evaporated. The residue was triturated with diethyl ether (2 x 100ml), and the combined organic phase dried (MgSO₄), filtered and evaporated. The residue was subjected to flash chromatography on silica gel, eluting with a mixture of ethyl acetate and hexane to afford the title compound (D10) as a white solid; yield 305mg.
δH (CDCl₃, 400MHz) 1.46(9H, s), 2.82 (2H, m), 3.12 (2H, m), 3.53 (4H, m), 4.02 (2H, s), 6.56 (1H, d), 6.82 (1H, d).

### Description 11

### 3-Acetyl-6-iodo-8-nitro-2,3,4,5-tetrahydro-1H-benzo[d]azepine (D11)

To a stirred solution of 3-acetyl-7-nitro-2,3,4,5-tetrahydro-1*H*-benzo[*d*]azepine [J. Heterocyl. Chem. (1971), **8(5),** 779-783] (2.71g, 11. 6mmol) in trifluoromethane-sulfonic acid (15ml) at room temperature was added *N*-iodosuccinimide (3.38g, 15mmol), portionwise over 1 hour, then the mixture stirred for 2 days. The mixture was poured onto ice and extracted with dichloromethane. After separating, the organic layer was washed with sodium thiosulfate solution, then brine and dried over magnesium sulfate. After filtration, the solvent was evaporated *in vacuo* to afford the title compound (D11), (2.74g, 69%).
δH (CDCl₃, 400MHz), 2.17,2.16 (3H, 2s), 3.10 (2H, m), 3.30 (2H, m), 3.65 (2H, m), 3.76 (2H, m), 8.00 (1H, 2d, J = 2.2Hz), 8.60 (1H, 2d, J = 2.2Hz).

### Description 12

### 6-Iodo-8-nitro-2,3,4,5-tetrahydro-1H-benzo[d]azepine (D12)

A mixture of 3-acetyl-6-iodo-8-nitro-2,3,4,5-tetrahydro-1*H*-benzo[*d*]azepine (D11) (2.74g, 7.59mmol) and hydrochloric acid (36%, 250ml) was heated at reflux for 2 days, cooled, filtered and evaporated to dryness. The residue was redissolved in water, treated with sodium bicarbonate and extracted with dichloromethane. The organic layer was dried, filtered and evaporated to afford the title compound (D12) (2.2g, 91%).
δH (DMSO-d6, 400MHz), 2.50 (4H, m), 3.04 (2H, m), 3.22 (2H, m), 8.02 (1H, d, J=2.4Hz), 8.40 (1H, d, J=2.4Hz).

### Description 13

### 6-Iodo-8-nitro-3-trifluoroacetyl-2,3,4,5-tetrabydro-1H-benzo[d]azepine (D13)

To a stirred solution of 6-iodo-8-nitro-2,3,4,5-tetrahydro-1*H*-benzo[*d*]azepine (D12) (2.2g, 6.9mmol) in dichloromethane (40ml), was added 2 equivalents of polymer supported Hunig's base [Argonault Tech.] (3.83mmol/g; 3.6g, 13.8mmol). After cooling to 5°C, trifluoroacetic anhydride (1.6g, 7.61mmol) was added dropwise, then stirred at room temperature for 18 hours. After filtration, the solution was washed with water then dried over magnesium sulfate. After filtration, the solvent was removed and the residue purified by chromatography on silica using 25% diethyl ether in hexane to afford the title compound (D13) (2.56g 90%).
δH (CDCl₃, 400MHz) 3.17 (2H, m), 3.38 (2H, m), 3.75 (4H, m) 8.01 (1H, m) 8.61 (1H, m).

### Description 14

### 6-Chloro-8-nitro-3-trifluoroacetyl-2,3,4,5-tetrahydro-1H-benzo[d]azepine (D14)

A mixture of 6-iodo-8-nitro-3-trifluoroacetyl-2,3,4,5-tetrahydro-1*H*-benzo[*d*]azepine (D13) (222mg, 0.5mmol), copper (I) chloride (362mg, 3.65mmol) and dimethyl formamide (5ml) were heated to 110°C for 18 hours with stirring. The solvent was removed and the residue purified by chromatography on silica using 25% diethyl ether in hexane to afford the title compound (D14), (130mg, 75%)
δH (CDCl₃, 400MHz) 3.15 (2H, m), 3.48 (2H, m), 3.76 (2H, m), 3.82 (2H, m), 7.95 (1H, m) 8.19 (1H, m).

### Description 15

### 6-Bromo-8-nitro-3-trifluoroacetyl-2,3,4,5-tetrahydro-1H-benzo[d]azepine (D15)

Prepared from 6-iodo-8-nitro-3-trifluoroacetyl-2,3,4,5-tetrahydro-1*H*-benzo[*d*]azepine (D13) and copper (I) bromide in a similar manner to Description 14, in 41% yield.
δH (CDCl₃, 400MHz) 3.18 (2H, m), 3.37 (2H, m), 3.60-3.85 (4H, m), 7.97-8.06 (1H, m), 8.36-8.37 (1H, m).

### Description 16

### 8-Amino-6-chloro-3-trifluoroacetyl-2,3,4,5-tetrahydro-1H-benzo[d]azepine (D16)

6-Chtoro-8-nitro-3-trifluoroacetyl-2,3,4,5-tetrahydro-1*H*-benzo[*d*]azepine (D14) (1.7g, 5.27mmol), was dissolved in methanol (20ml) and treated with 6 equivalents of titanium trichloride (30% solution in 2N HCl, 12.3ml, 5.27mmol) at room temperature with stirring, which was continued for a further hour. The reaction was treated with dropwise 30% hydrogen peroxide until the purple colour was extinguished, toluene added and the solvent removed. A saturated solution of sodium acetate in methanol was added until the pH rose to ∼5 then the solvent was removed. The residue was purified by chromatography on silica using 20-50% diethyl ether in hexane to afford the title compound (D16) (975mg, 63%).
δH (CDCl₃, 400MHz) 2.89 (2H, m), 3.11 (2H, m), 3.5 (2H br.s), 3.63-3.76 (4H, m), 6.38 (1H, m), 6.62 (1H, m).

### Description 17

### 8-Amino-6-bromo-3-trifluoroacetyl-2,3,4,5-tetrahydro-1H-benzo[d]azepine (D17)

Prepared from 6-bromo-8-nitro-3-trifluoroacetyl-2,3,4,5-tetrahydro-1*H*-benzo[*d*]azepine (D15) in a similar manner to Description 16, in 34% yield.
δH (CDCl₃, 400MHz) 2.0 (2H, br.s) 2.89 (2H, m), 3.16 (2H, m), 3.64-3.76 (4H, m), 6.42 (1H, m) 6.81 (1H, m).

### Description 18

### 7-Hydroxy-8-nitro-1,2,4,5-tetrahydro-benzo[d]azepine-3-carboxylic acid tert-butyl ester (D18)

7-Hydroxybenzazepine [WO 00/21951] (40g, 0.15 mol), was dissolved in glacial acetic acid (400ml) and added dropwise to a mixture of glacial acetic acid (200ml), acetic anhydride (20ml) and 70% nitric acid (16g). The reaction temperature was maintained at 10°C using an ice bath. Following addition, the mixture was allowed to warm to ambient and stirrer for a further 2 hours. The mixture was poured into ice/water (1 litre) and dichloromethane (2 litres) added. The organic phase was separated and neutralised to pH6 by addition of saturated sodium bicarbonate solution. The organic phase was separated, dried (MgSO₄) and purified by chromatography [Biotage Flash 75, 2Kg silica cartridge] using ethyl acetate and hexane as eluents to give the title compound (D 18) in 52% yield.
δH (CDCl₃, 400MHz) 1.48 (9H, s), 2.89 (4H, m), 3.56 (4H, m), 6.92 (1H, s), 7.84 (1H, s), 10.5 (1H, br.s).

### Description 19

### 7-Methoxy-8-nitro-1,2,4,5-tetrahydro-benzo[d]azepine-3-carboxylic acid tert-butyl ester (D19)

A mixture of 7-hydroxy-8-nitro-1,2,4,5-tetrahydro-benzo[*d*]azepine-3-carboxylic acid *tert*-butyl ester (D18) (49g, 0.16 mol), methyl iodide (12.4ml), potassium carbonate (27.4g) in N,N-dimethylformarnide (400ml) was stirred at room temperature for 16 hours. The mixture was poured into water (300ml) and extracted with diethyl ether (3 x 300ml), the combined organic phases washed with brine (50ml) and dried over MgSO₄ to afford the title compound (D19) in 96% yield.
δH (CDCl₃, 400MHz) 1.49 (9H, s), 2.88 (2H, m), 2.94 (2H, m), 3.57 (4H, m), 3.94 (3H, s), 6.84 (1H, s), 7.67 (1H, s).

### Description 20

### 8-Amino-7-methoxy-1,2,4,5-tetrahydro-benzo[d]azepine-3-carboxylic acid tert-butyl ester (D20)

A solution of 7-methoxy-8-nitro-1,2,4,5-tetrahydro-benzo[*d*]azepine-3-carboxylic acid *tert*-butyl ester (D19) (25g, 78mmol) in ethanol (700ml) was treated with 5% palladium on charcoal (5g) and hydrogenated at 50psi for 1 hour. The catalyst was removed by filtration under argon and the solution evaporated to give the title compound (D20) as a white solid in 96% yield.
δH (CDCl₃, 400MHz) 1.48 (9H, s), 2.76 (4H, m), 3.51 (4H, m), 3.65 (2H, brs), 3.82 (3H, s), 6.50 (1H, s), 6.55 (1H, s).

### Description 21

### 7-(3,5-Dichioro-2-methoxy-beazenesulfonylamino)-8-methoxy-1,2,4,5-tetrahydro-benzo[d]azepine-3-carboxylic acid tert-butyl ester (D21)

8-Amino-7-methoxy-1,2,4,5-tetrahydro-benzo[*d*]azepine-3-carboxylic acid *tert*-butyl ester (D20) (80mg) was dissolved in pyridine (0.5ml), cooled to 0°C and treated with 3,5-dichloro-2-methoxy-benzenesulfonyl chloride (110mg, 0.41mmol). After 16 hours, the solution was treated with methanol-water 1:1 (1ml) and evaporated. The residue was subjected to chromatography on silicagel, eluting with hexane and diethyl ether to give the title compound (D21) in 87% yield.
δH (CDCl₃, 400MHz) 1.46 (9H, s), 2.78 (4H, m), 3.48 (4H, m), 3.71 (3H, s), 3.87 (3H, s), 6.53 (1H, brs), 7.22 (1H, s), 7.46 (2H, app.s), 7.49 (1H, s).

### Description 22

### 7-(4-Bromo-2-trifluoromethoxy-benzenesulfonylamino)-6-chloro-1,2,4,5-tetrahydro-benzo[d]azepine-3-carboxylic acid tert butyl ester (D22)

A solution of 7-amino-6-chloro-1,2,4,5-tetrahydro-benzo[*d*]azepine-3-carboxylic acid tert-butyl ester (D10) (0.25mmol, 75mg) in dichloromethane (2ml) was treated with 4-bromo-2-trifluoromethoxy-benzenesulfonyl chloride (135mg 0.4mmol) in dichloromethane (2ml) and pyridine (0.2ml). After 14 hours, the solution was treated with water (0.5ml) and evaporated then the residue purified by flash chromatography on silicagel, eluting with diethyl ether and hexane to afford the title compound (D22) as a white solid.
δH (CDCl₃, 400MHz) 1.42 (9H, s), 2.85 (2H, m), 3.08 (2H, m), 3.48 (4H, m), 6.94 (1H, d), 7.33 (2H, m), 7.48 (2H, m), 7.85 (1H, d).

### Example 1

### 2,3,4,5-Tetrahydro-7-(3-trifluoromethyl)phenylsulfonamido-1H-benzo[d]azepine, hydrochloride (E1)

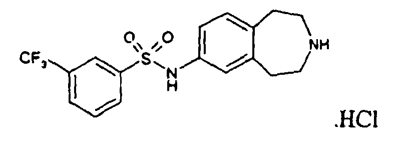

A suspension of 3-*t*-butyloxycarbonyl-7-(3-trifluoromethyl)phenylsulfonamido-2,3,4,5-tetrahydro-1*H*-benzo[*d*]azepine (D3) (910mg, 1.93 mmol) in hydrochloric acid (40ml, 4M in 1:1 water : 1,4-dioxane) was warmed to 60°C for I hour. The now clear solution was concentrated *in vacuo* and the residue crystallised from methanol/ether to afford the title compound (E1) as a pale brown solid (700mg, 89%).
δH (MeOH, 400MHz) 3.05-3.09 (4H, m), 3.22-3.26 (4H, m), 6.94 (1H, dd, J = 2.1, 8.1 Hz), 7.03 (1H, d, J = 2.1Hz), 7.12 (1H, d, J = 8.1 Hz), 7.73 (1H, app.t, J= 7.9Hz), 7.91 (1H, d, J = 7.8 Hz), 7.98 (1H, s), 8.03 (1H, d, J = 7.9Hz).
Found [MH]⁺ 371 (C₁₇H₁₇F₃N₂O₂S).

### Example 2

### 7-Phenylsulfonamido-2,3,4,5-tetrahydro-1H-benzo[d]azepine, hydrochloride (E2)

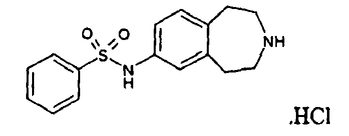

The title compound (E2) was prepared in 90% yield as described in Example 1, from 3-*t-*butyloxycarbonyl-7-phenylsulfonamido-2,3,4,5-tetrahydro-1*H*-benzo[*d*]azepine (D4).
δH (D6-DMSO, 400MHz) 2.98 (4H, br, s), 3.08 (4H, br, s), 6.87-6.93 (2H, m), 7.04 (1H, d, J = 8.1Hz), 7.53-7.63 (3H, m), 7.77 (1H, d, J= 7.3Hz), 930 (2H, br, s), 10.35 (1H, br, s).
Found [MH]⁺ 303 (C₁₆H₁₈N₂O₂S).

### Examples 3-5 (E3-E5)

Examples E3-E5 were prepared by a two step process, using the appropriate aryl sulfonyl chloride with 7-amino-3-t-butyloxycarbonyl-2,3,4,5-tetrahydro-1*H*-benzo[*d*]azepine (D2) in a similar manner to that described in Description 3, followed by a deprotection step in a similar manner to that described in Example 1.

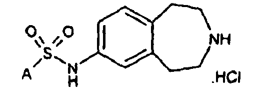

| Example | A | [MH]⁺ | Formula |
|---|---|---|---|
| E3 | 3-chlorophenyl | 337/339 | C₁₆H₁₇ClN₂O₂S |
| E4 | 5-bromo-2-thienyl | 387/389 | C₁₄H₁₅BrN₂O₂S₂ |
| E5 | 4-methylphenyl | 317 | C₁₇H₂₀N₂O₂S |

### Example 6

### 7-(4-Bromo-2-trifluoromethoxy)phenylsulfonamido-2,3,4,5-tetrahydro-1H-beozo[d]azepine (E6)

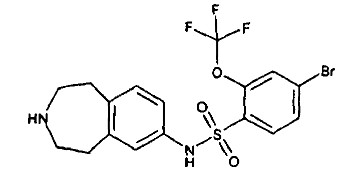

Prepared in an analogous procedure to E1 from 7-(4-bromo-2-trifluoromethoxy)phenylsulfonamido-3-*t*-butyloxycarbonyl-2,3,4,5-tetrahydro-1*H-*benzo[*d*]azepine (obtained in an analogous manner to that of D3 using 4-bromo-2-trifluoromethoxybenzenesulfonyl chloride). Found [MH]⁺ 465/467 (C₁₇H₁₆BrF₃N₂O₃S).

### Examples 7-9 (E7-E9)

Examples E7-E9 were prepared by a two step process, using the appropriate aryl sulfonyl chloride with 7-amino-3-t-butyloxycarbonyl-2,3,4,5-tetrahydro-1*H*-benzo[*d*]azepine (D2) in a similar manner to that described in Description 3, followed by a deprotection step in a similar manner to that described in Example 1.

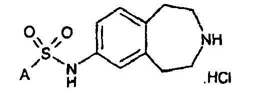

| | | | |
|---|---|---|---|
| E7 | 2,3-dichlorophenyl | 371/373 | C₁₆H₁₆Cl₂N₂O₂S |
| E8 | 3,5-dichloro-2-methoxyphenyl | 401/403 | C₁₇H₁₈Cl₂N₂O₃S |
| E9 | 4-bromo-2-ethylphenyl | 409/411 | C₁₈H₂₁BrN₂O₂S |

### Example 10

### 7-(4-Chloro-2-trifluoromethoxy)phenylsulfonamido-2,3,4,5-tetrahydro-1H-benzo[d]azepine (E10)

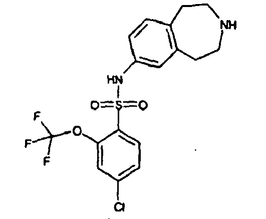

Prepared from 7-(4-bromo-2-trifluoromethoxy)phenylsulfonamido-3-trifluoroacetyl-2,3,4,5-tetrahydro-1*H*-benzo[*d*]azepine (obtained by treatment of E6 with trifluoroacetic anhydride in the presence of pyridine as a base) by reaction with copper (I) chloride in *N,N-*dimethylformamide at reflux, followed by removal of the trifluoroacetyl group using aqueous ammonia.
Found [MH]⁺ 421/423 (C₁₇H₁₆ClF₃N₂O₃S).

### Example 11

### 7-(4-Bromo-2-trifluoromethoxy)phenytsulfonamido-2,3,4,5-tetrabydro-1H-benzo[d]azepine, hydrochloride (E11)

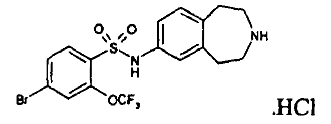

7-(4-Bromo-2-trifluoromethoxy)phenylsulfonamido-3-*t*-butyloxycarbonyl-2,3,4,5-tetrahydro-1*H-*benzo[*d*]azepine (D7) (30.2g, 53.5mmol) in dioxane (175ml) and 4N hydrochloric acid (175ml) was heated to 90°C with stirring. After 90 mins a solution was formed and the solvents were removed *in vacuo.* The residue was recrystallised from isopropanol to afford the title compound (E11)(23.6g, 88%).
δH (MeOd₄, 400MHz) 3.04 (4H, m), 3.22 (4H, m), 6.92 (1H, dd, J=2.4Hz, 8Hz), 7.01 (1H, d, J=2.4Hz), 7.08 (1H,d, J=8Hz), 7.64, (2H, m), 7.88, (1H, d, J=8.8Hz).
Found [MH]⁺ 465/467 (C₁₇H₁₆BrF₃N₂O₃S)
m.p. 231 - 233 °C

### Example 12

### 7-(4-Chloro-2-trifluoromethoxy)phenylsulfonamido-2,3,4,5-tetrahydro-1H benzo[d]azepine, hydrochloride (E12)

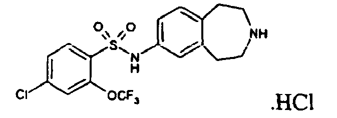

To a stirred suspension of 7-(4-chloro-2-trifluoromethoxy)phenylsulfonamido-3-trifluoroacetyl-2,3,4,5-tetrahydro-1*H*-benzo[*d*]azepine (D9) (30.3g, 58.6mmol) in methanol (750ml) was added 32% ammonia solution (75ml) and the resulting solution stirred at room temperature for 24h. The reaction mixture was then evaporated *in vucuo,* then the residue was suspended in toluene (800ml) and solvent evaporated *in vacuo.* The residue was again suspended in toluene (800ml) and solvent evaporated *in vacuo.* To the resulting colourless residue was added diethyl ether (400ml) and the mixture stirred for 30 minutes before being filtered and dried *in vacuo* at 50 °C. To a suspension of this material in dichloromethane (250ml) was added 1M HCl/diethyl ether (123ml) followed by methanol (50ml). The resulting solution was concentrated to an oil and to this added dichloromethane (100ml)/diethyl ether (300ml) to give fine white crystals. The crystals were filtered, washed with dichloromethane/diethyl ether (1:3) (2x75ml) and diethyl ether (2x75ml) before being dried *in vacuo.* The material was then recrystallised from *iso*propanol to afford the desired compound as a white solid (21.8g, 81%).
δH (DMSO-d₆, 400MHz) 2.99-3.01 (4H, m), 3.08 (4H, br s), 6.86 (1H, dd, J = 8.1Hz, 2.3Hz), 6.94 (1H, d, J = 2.2Hz), 7.07 (1H, d, J = 8.2Hz), 7.66-7.70 (2H, m), 7.97 (1H, d, J = 8.5Hz), 9.37 (2H, br s), 10.70 (1H, br s).
Found [MH]⁺ 420/422 (C₁₇H₁₆ClF₃N₂O₃S)
m.p. 210 - 211 °C.

### Examples 13-15 (E13-E15)

Examples E13-E15 were prepared by treatment of the appropriate trifluoroacetyl protected aminobenzo[*d*]azepine derivative (D16 for E13 and E14 and D 17 for E15) with the required arylsulfonyl chloride in an analogous manner to the process described in D8, followed by treatment of the product with aqueous ammonia (2M), and subsequent purified by recrystallisation of their respective hydrochlorides.

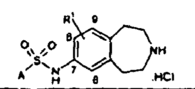

| Example | R¹ | A | [MH]⁺ | Formula |
|---|---|---|---|---|
| E13 | 9-Cl | phenyl | 337 | C₁₆H₁₇ClN₂O₂S |
| E14 | 9-Cl | (3-trifluoromethyl)phenyl | 405 | C₁₇H₁₆ClF₃N₂O₂S |
| E15 | 9-Br | phenyl | 381/383C | C₁₆H₁₇BrN₂O₂S |

### Example 16

### 7-(4-Bromo-2-triftuoromethoxy-benzenesulfonylamino)-6-chloro-1,2,4,5-tetrahydro-benzo[d]azepine Hydrochloride (E16)

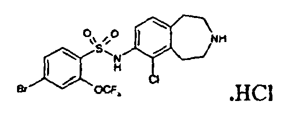

Prepared from 7-(4-bromo-2-trifluoromethoxy-benzenesulfonylamino)-6-chloro-1,2,4,5-tetrahydro-benzo[*d*]azepine-3-carboxylic acid *tert* butyl ester (D22) by an analogous method to that described for Example 1.
δH (MeOH-d₄, 400MHz) 3.15-3.47 (8H, m), 7.15 (1H, d), 7.39 (1H, d), 7.65, (2H, m), 7.89, (1H, d).
δH (MeOH-d₄, 400MHz) 3.15-3.47 (8H, m), 7.17 (1H, d), 7.37 (1H, d), 7.65, (2H, m), 7.82, (1H, d).
Found [MH]⁺ 499, 501, 503 (C₁₇H₁₅BrClF₃N₂O₃S)

### Example 17

### 3,5-Dichloro-2-methoxy-N-(8-methoxy-2,3,4,5-tetrahydro-1H-benzo[d]azepin-7-yl)-benzenesulfonamide hydrochloride (E17)

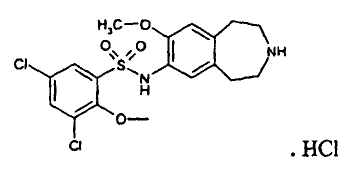

Prepared from 7-(3,5-dichloro-2-methoxy-benzenesulfonylamino)-8-methoxy-1,2,4,5-tetrahydro-benzo[*d*]azepine-3-carboxylic acid *tert*-butyl ester (D21) by an analogous method to that described for Example 1.
δH (MeOD, 400MHz) 2.99 (m, 4H), 3.14 (m, 4H), 3.55 (3H, s), 3.74 (3H, s), 6.70 (1H, s), 7.18 (1H, s), 7.52 (1H, s), 7.57 (1H, s)
Found [MH]⁺ 431, 433,435 (C₁₈H₂₀Cl₂N₂O₄S)

### Pharmacological data

Compounds can be tested following the procedures outlined in WO98/27081.
The compounds of Examples E1-E17 were tested and showed good affinity for the 5-HT₆ receptor, having pKi values > 8 at human cloned 5-HT₆ receptors. More specifically, the compounds of Examples 6 and 10 demonstrated pKi values > 8.5 at human cloned 5-HT₆ receptors.

## Claims

1. A compound of formula (I) or a pharmaceutically acceptable salt thereof: 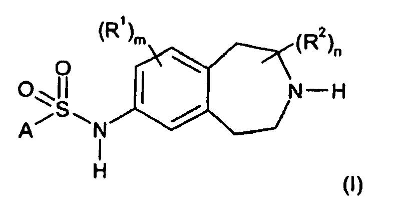 wherein:
R¹ represents hydrogen, halogen, hydroxy, cyano, nitro, trifluoromethyl, trifluoromethoxy, C₁₋₆ alkyl, trifluoromethanesulfonyloxy, pentafluoroethyl, C₁₋₆ alkoxy, arylC₁₋₆ alkoxy, C₁₋₆ alkylthio, C₁₋₆ alkoxyC₁₋₆ alkyl, C₃₋₇ cycloalkylC₁₋₆ alkoxy, C₁₋₆ alkanoyl, C₁₋₆ alkoxycarbonyl, C₁₋₆ alkylsulfonyl, C₁₋₆ alkylsulfinyl, C₁₋₆ alkylsulfonyloxy, C₁₋₆ alkylsulfonylC₁₋₆ alkyl, arylsulfonyl, arylsulfonyloxy, arylsulfonylC₁₋₆ alkyl, C₁₋₆ alkylsulfonamido, C₁₋₆ alkylamido, C₁₋₆ alkylsulfonamidoC₁₋₆ alkyl, C₁₋₆ alkylamidoC₁₋₆ alkyl, arylsulfonamido, arylcarboxamido, arylsulfonamidoC₁₋₆ alkyl, arylcarboxamidoC₁₋₆ alkyl, aroyl, aroylC₁₋₆ alkyl, arylC₁₋₆ alkanoyl, or a group CONR³R⁴ or SO₂NR³R⁴, wherein R³ and R⁴ independently represent hydrogen or C₁₋₆ alkyl or together may be fused to form a 5- to 7- membered aromatic or non-aromatic heterocyclic ring optionally interrupted by an O or S atom;
R² represents hydrogen or C₁₋₆ alkyl;
m represents an integer from 1 to 3;
n represents an integer from 1 to 4;
A represents phenyl, naphthyl or a monocyclic or bicyclic heteroaryl group each of which may be optionally substituted by one or more substituents which may be the same or different, and which are selected from those defined for R¹;
or solvates thereof;
with the proviso that when A represents phenyl substituted at the 4-position, said substituent is not trifluoromethyl, trifluoromethoxy, C₃₋₆ alkyl or C₁₋₆ alkoxy.

2. A compound of formula (I) as defined in claim 1 which is
2,3,4,5-Tetrahydro-7-(3-trifluoromethyl)phenylsulfonamido-1*H*-benzo[*d*]azepine; 7-Phenylsulfonamido-2,3,4,5-tetrahydro-1*H*-benzo[*d*]azepine;
2,3,4,5-Tetrahydro-7-(3-chloro)phenylsulfonamido-1*H*-benzo[*d*]azepine;
2,3,4,5-Tetrahydro-7-(5-bromo-2-thienyl)sulfonamido-1*H*-benzo[*d*]azepine;
2,3,4,5-Tetrahydro-7-(4-methyl)phenylsulfonamido-1*H*-benzo[*d*]azepine;
7-(4-Bromo-2-trifluoromethoxy)phenylsulfonamido-2,3,4,5-tetrahydro-1*H-*benzo[*d*]azepine;
2,3,4,5-Tetrahydro-7-(2,3-dichloro)phenylsulfonamido-1*H-*benzo[*d*]azepine;
2,3,4,5-Tetrahydro-7-(3,5-dichloro-2-methoxy)phenylsulfonamido-1*H*-benzo[*d*]azepine;
2,3,4,5-Tetrahydro-7-(4-bromo-2-ethyl)phenylsulfonamido-1*H*-benzo[*d*]azepine;
7-(4-Chloro-2-trifluoromethoxy)phenylsulfonamido-2,3,4,5-tetrahydro-1*H-*benzo[*d*]azepine;
7-(Benzenesulfonylamino)-9-chloro-1,2,4,5-tetrahydro-benzo[*d*]azepine;
7-(3-trifluoromethyl-benzenesulfonylamino)-9-chloro-1,2,4,5-tetrahydro-benzo[*d*]azepine;
7-(Benzenesulfonylamino)-9-bromo-1,2,4,5-tetrahydro-benzo[*d*]azepine;
7-(4-Bromo-2-trifluoromethoxy-benzenesulfonylamino)-6-chloro-1,2,4,5-tetrahydro-benzo[*d*]azepine;
3,5-Dichloro-2-methoxy-*N*-(8-methoxy-2,3,4,5-tetrahydro-1*H*-benzo[*d*]azepin-7-yl)-benzenesulfonamide;
or a pharmaceutically acceptable salt thereof.

3. A pharmaceutical composition which comprises a compound according to claim 1 or claim 2 and a pharmaceutically acceptable carrier or excipient.

4. A compound according to claim 1 or claim 2 for use in therapy.

5. A compound according to claim 1 or claim 2 for use in the treatment of depression, anxiety, Alzheimers disease, age related cognitive decline, ADHD, obesity, mild cognitive impairment and schizophrenia.

6. The use of a compound of formula (I) as defined in claim 1 or claim 2 or a pharmaceutically acceptable salt thereof in the manufacture of a medicament for the treatment or prophylaxis of depression, anxiety, Alzheimers disease, age related cognitive decline, ADHD, obesity, mild cognitive impairment and schizophrenia.

## Patentansprüche

1. Verbindung der Formel (I) oder ein pharmazeutisch verträgliches Salz davon: 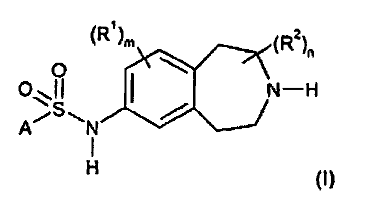 wobei:
R¹ Wasserstoff, Halogen, Hydroxy, Cyano, Nitro, Trifluormethyl, Trifluormethoxy, C₁₋₆-Alkyl, Trifluormethansulfonyloxy, Pentafluorethyl, C₁₋₆-Alkoxy, Aryl-C₁₋₆-alkoxy, C₁₋₆-Alkylthio, C₁₋₆-Alkoxy-C₁₋₆-alkyl, C₃₋₇-Cycloalkyl-C₁₋₆-alkoxy, C₁₋₆-Alkanoyl, C₁₋₆-Alkoxycarbonyl, C₁₋₆-Alkylsulfonyl, C₁₋₆-Alkylsulfinyl, C₁₋₆-Alkylsulfonyloxy, C₁₋₆-Alkylsulfonyl-C₁₋₆-alkyl, Arylsulfonyl, Arylsulfonyloxy, Arylsulfonyl-C₁₋₆-alkyl, C₁₋₆-Alkylsulfonamido, C₁₋₆-Alkylamido, C₁₋₆-Alkylsulfonamido-C₁₋₆-alkyl, C₁₋₆₋Alkylamido-C₁₋₆-alkyl, Arylsulfonylamido, Arylcarboxamido, Arylsulfonamido-C₁₋₆₋alkyl, Arylcarboxamido-C₁₋₆-alkyl, Aroyl, Aroyl-C₁₋₆-alkyl, Aryl-C₁₋₆-alkanoyl oder einen Rest CONR³R⁴ oder SO₂NR³R⁴ darstellt, wobei R³ und R⁴ unabhängig voneinander Wasserstoff oder C₁₋₆-Alkyl darstellen oder aneinander gebunden sein können, um einen 5- bis 7-gliedrigen aromatischen oder nicht aromatischen heterocyclischen Ring, gegebenenfalls von einem O- oder S-Atom unterbrochen, zu bilden;
R² Wasserstoff oder C₁₋₆-Alkyl darstellt;
m eine ganze Zahl von 1 bis 3 darstellt;
n eine ganze Zahl von 1 bis 4 darstellt;
A Phenyl, Naphthyl oder einen monocyclischen oder bicyclischen Heteroarylrest darstellt, welcher jeweils gegebenenfalls mit einem oder mehreren Substituenten, welche gleich oder verschieden sein können und welche aus den für R¹ definierten Resten ausgewählt sind, substituiert sein kann;
oder Solvate davon;
mit der Maßgabe, dass wenn A Phenyl darstellt, das an der 4-Position substituiert ist, der Substituent nicht Trifluormethyl, Trifluormethoxy, C₃₋₆-Alkyl oder C₁₋₆-Alkoxy ist.

2. Verbindung der Formel (I) gemäß Anspruch 1, welche ist:
2,3,4,5-Tetrahydro-7-(3-trifluormethyl)phenylsulfonamido-1*H*-benz[d]azepin;
7-Phenylsulfonamido-2,3,4,5-tetrahydro-1*H*-benz[d]azepin;
2,3,4,5-Tetrahydro-7-(3-chlor)phenylsulfonamido-1*H*-benz[d]azepin;
2,3 ,4,5-Tetrahydro-7-(5-brom-2-thienyl)sulfonamido-1*H*-benz[d]azepin;
2,3,4,5-Tetrahydro-7-(4-methyl)phenylsulfonamido-1*H*-benz[d]azepin;
7-(4-Brom-2-trifluormethoxy)phenylsulfonamido-2,3,4,5-tetrahydro-1*H*-benz[d]azepin;
2,3,4,5-Tetrahydro-7-(2,3-dichlor)phenylsulfonamido-1*H*-benz[d]azepin;
2,3,4,5-Tetrahydro-7-(3,5-dichlor-2-methoxy)phenylsulfonamido-1*H*-benz[d]azepin;
2,3,4,5-Tetrahydro-7-(4-brom-2-ethyl)phenylsulfonamido-1*H*-benz[d]azepin;
7-(4-Chlor-2-trifluormethoxy)phenylsulfonamido-2,3,4,5-tetrahydro-1*H*-benz[d]azepin;
7-(Benzolsulfonylamino)-9-chlor-1,2,4,5-tetrahydrobenz[d]azepin;
7-(3-Trifluormethylbenzolsulfonylamino)-9-chlor-1,2,4,5-tetrahydrobenz[d]azepin;
7-(Benzolsulfonylamino)-9-brom-1,2,4,5-tetrahydrobenz[d]azepin;
7-(4-Brom-2-trifluormethoxybenzolsulfonylamino)-6-chlor-1,2,4,5-tetrahydrobenz-[d]azepin;
3,5-Dichlor-2-methoxy-*N*-(8-methoxy-2,3,4,5-tetrahydro-1*H*-benz[*d*]azepin-7-yl)benzolsulfonamid;
oder ein pharmazeutisch verträgliches Salz davon.

3. Arzneimittel, welches eine Verbindung gemäß Anspruch 1 oder Anspruch 2 und einen pharmazeutisch verträglichen Träger oder Exzipienten umfasst.

4. Verbindung gemäß Anspruch 1 oder Anspruch 2 zur Verwendung in der Therapie.

5. Verbindung gemäß Anspruch 1 oder Anspruch 2 zur Verwendung in der Behandlung von Depression, Angst, Alzheimer Krankheit, mit dem Alter verbundenem kognitiven Verfall, ADHD, Fettsucht, schwacher kognitiver Verschlechterung und Schizophrenie.

6. Verwendung einer Verbindung der Formel (I) gemäß Anspruch 1 oder Anspruch 2 oder eines pharmazeutisch verträglichen Salzes davon zur Herstellung eines Medikaments zur Behandlung oder Prophylaxe von Depression, Angst, Alzheimer Krankheit, mit dem Alter verbundenem kognitiven Verfall, ADHD, Fettsucht, schwacher kognitiver Verschlechterung und Schizophrenie.

## Revendications

1. Composé de formule (I) ou un de ses sels pharmaceutiquement acceptables : 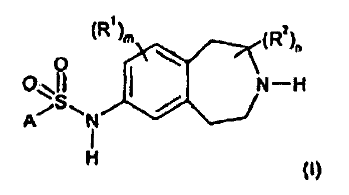 dans laquelle :
R¹ représente un atome d'hydrogène, un atome d'halogène, un groupe hydroxy, cyano, nitro, trifluorométhyle, trifluorométhoxy, alkyle en C₁ à C₆, trifluorométhane-sulfonyloxy, pentafluoréthyle, alkoxy en C₁ à C₆, aryl(alkoxy en C₁ à C₆), alkylthio en C₁ à C₆, (alkoxy en C₁ à C₆) (alkyle en C₁ à C₆), (cycloalkyle en C₃ à C₇) (alkoxy en C₁ à C₆), alkanoyle en C₁ à C₆, (alkoxy en C₁ à C₆)carbonyle, alkylsulfonyle en C₁ à C₆, alkylsulfinyle en C₁ à C₆, alkylsulfonyloxy en C₁ à C₆, (alkyle en C₁ à C₆) sulfonyle-(alkyle en C₁ à C₆), arylsulfonyle, arylsulfonyloxy, arylsulfonyl (alkyle en C₁ à C₆), alkylsulfonamido en C₁ à C₆, alkylamido en C₁ à C₆, (alkyle en C₁ à C₆) sulfonamido (alkyle en C₁ à C₆), (alkyle en C₁ à C₆) amido (alkyle en C₁ à C₆), arylsulfonamido, arylcarboxamido, arylsulfonamido(alkyle en C₁ à C₆), arylcarboxamido(alkyle en C₁ à C₆), aroyle, aroyl (alkyle en C₁ à C₆), aryl (alkanoyle en C₁ à C₆) ou un groupe CONR³R⁴ ou SO₂NR³R⁴, dans lesquels R³ et R⁴ représentent indépendamment un atome d'hydrogène ou un groupe alkyle en C₁ à C₆ ou bien, conjointement, peuvent être condensés pour former un noyau hétérocyclique penta- à heptagonal aromatique ou non aromatique facultativement interrompu par un atome de O ou S ;
R² représente un atome d'hydrogène ou un groupe alkyle en C₁ à C₆ ;
m représente un nombre entier de 1 à 3 ;
n représente un nombre entier de 1 à 4 ;
A représente un groupe phényle, un groupe naphtyle ou un groupe hétéroaryle monocyclique ou bicyclique, chacun pouvant être facultativement substitué avec un ou plusieurs substituants qui peuvent être identiques ou différents et qui sont choisis parmi ceux définis pour R¹ ;
ou ses produits de solvatation ;
sous réserve que, lorsque A représente un groupe phényle substitué en position 4, ledit substituant ne soit pas un groupe trifluorométhyle, trifluorométhoxy, alkyle en C₃ à C₆ ou alkoxy en C₁ à C₆.

2. Composé de formule (I) telle que définie dans la revendication 1, qui est
la 2,3,4,5-tétrahydro-7-(3-trifluorométhyl)phénylsulfonamido-*1H*-benzo[*d*]azépine ;
la 7-phénylsulfonamido-2,3,4,5-tétrahydro-*1H*-benzo[*d*]azépine ;
la 2,3,4,5-tétrahydro-7-(3-chloro)phénylsulfonamido-*1H*-benzo-[*d*]azépine ;
la 2,3,4,5-tétrahydro-7-(5-bromo-2-thiényl)sulfonamido-*1H*-benzo-[*d*]azépine ;
la 2,3,4,5-tétrahydro-7-(4-méthyl)phénylsulfonamido-*1H*-benzo-[*d*]azépine ;
la 7-(4-bromo-2-trifluorométhoxy)phénylsulfonamido-2,3,4,5-tétrahydro-1H-benzo[d]azépine ;
la 2,3,4,5-tétrahydro-7-(2,3-dichloro)phénylsulfonamido-*1H-*benzo [d] azépine ;
la 2,3,4,5-tétrahydro-7-(3,5-dichloro-2-méthoxy)phénylsulfonamido-*1H*-benzo[*d*]azépine ;
la 2,3,4,5-tétrahydro-7-(4-bromo-2-éthyl)phénylsulfonamido-*1H*-benzo[*d*]azépine ;
la 7-(4-chloro-2-trifluorométhoxy)phénylsulfonamido-2,3,4,5-tétrahydro-*1H*-benzo[*d*]azépine ;
la 7-(benzènesulfonylamino)-9-chloro-1,2,4,5-tétrahydrobenzo-[*d*]azépine ;
la 7-(3-trifluorométhyl-benzènesulfonylamino)-9-chloro-1,2,4,5-tétrahydrobenzo[d]azépine ;
la 7-(benzènesulfonylamino)-9-bromo-1,2,4,5-tétrahydrobenzo-[*d*] azépine ;
la 7-(4-bromo-2-trifluorométhoxy-benzènesulfonylamino)-6-chloro-1,2,4,5-tétrahydrobenzo[Id]azépine ;
le 3,5-dichloro-2-méthoxy-*N*-(8-méthoxy-2,3,4,5-tétrahydro-1*H-*benzo[*d*]azépine-7-yl)-benzènesulfonamide ;
ou un de leurs sels pharmaceutiquement acceptables.

3. Composition pharmaceutique qui comprend un composé suivant la revendication 1 ou la revendication 2 et un support ou excipient pharmaceutiquement acceptable.

4. Composé suivant la revendication 1 ou la revendication 2, destiné à être utilisé en thérapeutique.

5. Composé suivant la revendication 1 ou la revendication 2, destiné à être utilisé dans le traitement de la dépression, de l'anxiété, de la maladie d'Alzheimer, du déclin cognitif dû à l'âge, de l'ADHD, de l'obésité, de l'altération cognitive faible et de la schizophrénie.

6. Utilisation d'un composé de formule (I) tel que défini dans la revendication 1 ou la revendication 2 ou d'un de ses sels pharmaceutiquement acceptables dans la production d'un médicament destiné au traitement ou à la prophylaxie de la dépression, de l'anxiété, de la maladie d'Alzheimer, du déclin cognitif dû à l'âge, de l'ADHD, de l'obésité, de l'altération cognitive faible et de la schizophrénie.
